# EUROPEAN PATENT APPLICATION

(11) **EP 4 603 413 A1**
(43) Date of publication of application: **20.08.2025**
(21) Application number: 24305237.0
(22) Date of filing: 13.02.2024
(51) Int. Cl.: B65D 75/38, A61L 2/00, B65D 81/32

(54) **MULTI-CHAMBER BAG**

(71) Applicant: Becton Dickinson France, 38800 Le Pont-de-Claix (FR)
(72) Inventor: LE LOC'H, Clémentine, 38240 Meylan (FR); EYMERY, Anaïs, 38450 Saint-Georges-de-Commiers (FR)
(74) Representative: Germain Maureau

(57) **Abstract**

A bag, including a first sheet including a porous portion and a gas-impervious portion, the first sheet having: a first surface, a second surface opposite the first surface, a first end, a second end opposite the first end, and a longitudinal axis extending between the first and second ends; a second sheet attached to the first surface of the first sheet to define a first compartment between the first and second sheets; and a third sheet attached to the second surface of the first sheet to define a second compartment between the first and third sheets, wherein the second compartment is longitudinally offset from the first compartment.

## Description

### FIELD OF THE INVENTION

The present disclosure relates generally to containers for sterilizing items, and more particularly, containers with multiple compartments that are independently accessible.

### BACKGROUND OF THE INVENTION

Existing methods of sterilizing items, such as medical devices, containers, or components thereof, typically involve a multi-step process in which the item(s) to be sterilized are placed in a container or bag formed of a porous material, such as paper or a nonwoven sheet material, such as Tyvek. The bag and the contents therein may then be treated with a sterilizing gas, such as ethylene oxide, steam sterilization, or irradiated with other sterilizing energy, radiation, and/or agents. Once sterilization is completed, the bag containing the sterilized items may be inserted into an additional outer container or bag comprising a non-porous material. The bag with the sterilized items are then typically sealed in the outer bag until subsequently removed for use.

The use of multiple steps and multiple containers or bags can be cumbersome, time consuming, and costly, rendering such processes less than desirable. In addition, the resulting multi-bag final packaging tends to be heavier than desirable, utilizes larger quantities of material, and are thus more labor intensive to produce. Moreover, such conventional packaging generally does not provide for sufficient control of residual sterilization agents or moisture in the package, which can adversely affect the sterilized items.

The present disclosure provides improved systems, devices, and methods of use thereof for packaging, sterilizing, storing, and/or transporting sterilizable items, such as medical devices and components thereof.

### SUMMARY OF THE INVENTION

The present disclosure provides a bag, including a first sheet including a porous portion and a gas-impervious portion, the first sheet having: a first surface, a second surface opposite the first surface, a first end, a second end opposite the first end, and a longitudinal axis extending between the first and second ends; a second sheet attached to the first surface of the first sheet to define a first compartment between the first and second sheets; and a third sheet attached to the second surface of the first sheet to define a second compartment between the first and third sheets, wherein the second compartment is longitudinally offset from the first compartment.

The bag may include a first opening between the first and second sheets, where the first opening may be located adjacent the first end of the first sheet and in fluid communication with the first compartment. The bag may include a second opening between the first and third sheets, where the second opening may be located adjacent the second end of the first sheet and in fluid communication with the second compartment.

The bag may include a first seal joining the first sheet and the second sheet, where the first seal may extend substantially transverse to the longitudinal axis and may be longitudinally positioned between the first opening and the second end of the first sheet. The second opening may be longitudinally positioned between the first seal and the second end of the first sheet. The bag may include a second seal joining the first sheet and the third sheet, where the second seal may extend substantially transverse to the longitudinal axis and may be longitudinally positioned between the second opening and the first end of the first sheet.

The first opening may be longitudinally positioned between the second seal and the first end of the first sheet. The first sheet may define a length between the first end and second end, and the length may be greater than a length of the second sheet. The length of the first sheet may be greater than a length of the third sheet.

A method of sterilizing an article is provided, including providing a bag including a first sheet including a porous portion and a gas-impervious portion, the first sheet having: a first surface, a second surface opposite the first surface, a first end, a second end opposite the first end, and a longitudinal axis extending between the first and second ends; a second sheet attached to the first surface of the first sheet to define a first compartment between the first and second sheets; and a third sheet attached to the second surface of the first sheet to define a second compartment between the first and third sheets, wherein the second compartment is longitudinally offset from the first compartment. The method also includes placing an item in the first compartment of the bag; sealing the second sheet to the first sheet; introducing a sterilizing agent into the second compartment such that the sterilizing agent passes through the porous portion of the first sheet and into the first compartment; positioning a charcoal element into the second compartment; and sealing the third sheet to the first sheet.

The method may include creating a vacuum in the second compartment prior to sealing the third sheet to the first sheet. The sterilizing agent may be at least one of ethylene oxide or steam.

A method of assembling a bag is provided, including positioning a first sheet in contact with least a portion of a second sheet, wherein the first sheet includes a porous portion and a gas-impervious portion, the first sheet further including: a first surface, a second surface opposite the first surface, a first end, a second end opposite the first end, and a longitudinal axis extending between the first and second ends; forming a first seal between the first surface of the first sheet and the second sheet, wherein the first seal extends substantially transverse to the longitudinal axis; moving the second sheet such that at least a portion of the second sheet does not overlap the first sheet; positioning a third sheet in contact with at least a portion of the second surface of the first sheet second sheet; forming a second seal between the first surface of the first sheet and the second sheet, wherein the first seal extends substantially transverse to the longitudinal axis; positioning the first sheet, second sheet, and third sheet to substantially overlay each other; forming a third seal between the first, second, and third sheets, wherein the third seal extends substantially parallel to the longitudinal axis; and forming a fourth seal between the first, second, and third sheets, wherein the fourth seal extends substantially parallel to the longitudinal axis, wherein the first, third, and fourth seals define a first compartment between the first and second sheets, and wherein the second, third, and fourth seals define a second compartment between the first and third sheets.

The first compartment may be longitudinally offset from the second compartment. The first compartment may be accessible through a first opening between the first and second sheets adjacent to the first end of the first sheet. The second compartment may be accessible through a second opening between the first and third sheets adjacent to the second end of the first sheet. The first seal may be longitudinally positioned between the second opening and the second seal. The second seal may be longitudinally positioned between the first opening and the first seal. The first sheet may define a length between the first end and second end, and the length may be greater than a length of the second sheet. The length of the first sheet may be greater than a length of the third sheet.

### BRIEF DESCRIPTION OF THE DRAWINGS

A more complete understanding of the present disclosure, and the attendant advantages and features thereof, will be more readily understood by reference to the following detailed description when considered in conjunction with the accompanying drawings, wherein:
FIG. 1 is an exploded perspective assembly view of an example of a bag constructed in accordance with the principles of the present disclosure;
FIGS. 2A-2C illustrate examples of configurations of porous portions of a bag constructed in accordance with the principles of the present disclosure;
FIGS. 3A-3B are side cross-sectional views of examples of a bag constructed in accordance with the principles of the present disclosure;
FIG. 4 is another side cross-sectional view of an example of a bag constructed in accordance with the principles of the present disclosure;
FIG. 5A-5B are additional side cross-sectional views of examples of a bag constructed in accordance with the principles of the present disclosure;
FIG. 6 is an illustration of a step of an assembly process to construct an example of a bag according to the principles of the present disclosure;
FIG. 7 is an illustration of another step of an assembly process to construct an example of a bag according to the principles of the present disclosure;
FIG. 8 is an illustration of another step of an assembly process to construct an example of a bag according to the principles of the present disclosure; and
FIG. 9 is an illustration of another step of an assembly process to construct an example of a bag according to the principles of the present disclosure.

### DETAILED DESCRIPTION OF THE INVENTION

The following description is provided to enable those skilled in the art to make and use the described aspects contemplated for carrying out the invention. Various modifications, equivalents, variations, and alternatives, however, will remain readily apparent to those skilled in the art. Any and all such modifications, variations, equivalents, and alternatives are intended to fall within the spirit and scope of the present invention.

As used herein, the singular form of "a", "an", and "the" include plural referents unless the context clearly dictates otherwise.

Spatial or directional terms, such as "left", "right", "inner", "outer", "above", "below", and the like, relate to the embodiments or aspects as shown in the drawing figures and are not to be considered as limiting, as the embodiments or aspects can assume various alternative orientations.

All numbers used in the specification and claims are to be understood as being modified in all instances by the term "about". By "about" is meant within plus or minus twenty-five percent of the stated value. However, this should not be considered as limiting to any analysis of the values under the doctrine of equivalents.

Unless otherwise indicated, all ranges or ratios disclosed herein are to be understood to encompass the beginning and ending values and any and all subranges or subratios subsumed therein. For example, a stated range or ratio of "1 to 10" should be considered to include any and all subranges or subratios between (and inclusive of) the minimum value of 1 and the maximum value of 10; that is, all subranges or subratios beginning with a minimum value of 1 or more and ending with a maximum value of 10 or less. The ranges and/or ratios disclosed herein represent the average values over the specified range and/or ratio.

The terms "first", "second", and the like are not intended to refer to any particular order or chronology, but refer to different conditions, properties, or elements.

All documents referred to herein are "incorporated by reference" in their entirety.

The term "at least" is synonymous with "greater than or equal to".

As used herein, "at least one of' is synonymous with "one or more of'. For example, the phrase "at least one of A, B, or C" means any one of A, B, or C, or any combination of any two or more of A, B, or C. For example, "at least one of A, B, and C" includes A alone; or B alone; or C alone; or A and B; or A and C; or B and C; or all of A, B, and C.

The word "comprising" and "comprises", and the like, does not exclude the presence of elements or steps other than those listed in any claim or the specification as a whole. In the present specification, "comprises" means "includes" and "comprising" means "including".

As used herein, the terms "parallel" or "substantially parallel" mean a relative angle as between two objects (if extended to theoretical intersection), such as elongated objects and including reference lines, that is from 0° to 5°, or from 0° to 3°, or from 0° to 2°, or from 0° to 1°, or from 0° to 0.5°, or from 0° to 0.25°, or from 0° to 0.1°, inclusive of the recited values.

As used herein, the terms "perpendicular", "transverse", "substantially perpendicular", or "substantially transverse" mean a relative angle as between two objects at their real or theoretical intersection is from 85° to 90°, or from 87° to 90°, or from 88° to 90°, or from 89° to 90°, or from 89.5° to 90°, or from 89.75° to 90°, or from 89.9° to 90°, inclusive of the recited values.

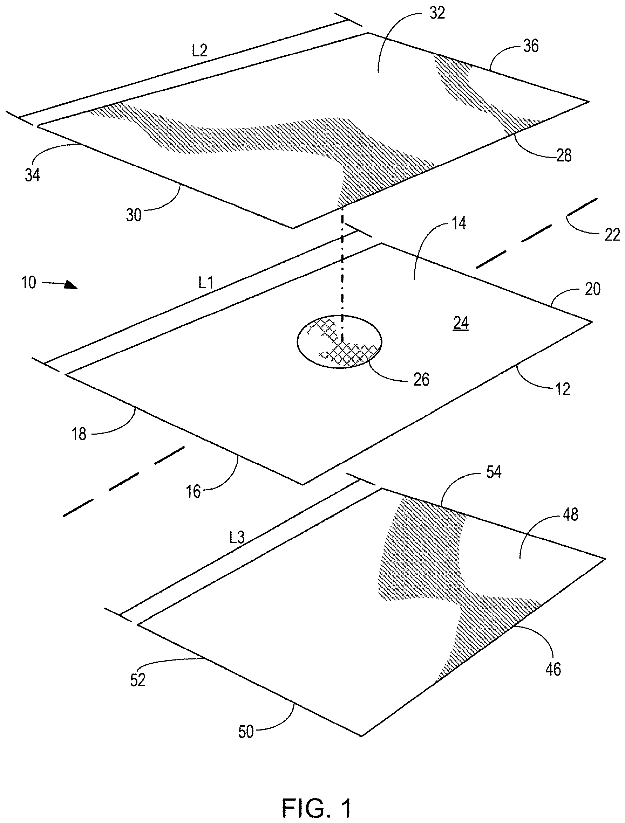

The present disclosure provides systems, devices, and methods of use thereof for packaging, sterilizing, storing, and/or transporting sterilizable items, such as medical devices. Referring now to the figures in which like reference designations refer to like elements, examples of a bag 10 and aspects and components thereof are shown in FIGS. 1-5.

The bag 10 may include, define, or be constructed from a plurality of sheets of flexible material to form or define a plurality of compartments therein. For example, the bag 10 may include a first sheet 12 of flexible material. The first sheet 12 may include or define a first surface 14, a second surface 16 opposite the first surface 14, a first end 18, a second end 20 opposite the first end 18, and a longitudinal axis 22 extending from the first end 18 to the second end 20. The first sheet 12 may define a length L1 as measured between the first and second ends 18, 20 along the longitudinal axis 22.

The first sheet 12 may define or include a substantially non-porous, gas-impervious portion 24 and a gas permeable, porous portion 26. "Gas-impervious" refers to a portion with a low permeability to gases. The permeability of the gas-impervious portion 24 may depend on a number of different factors, including but not limited to the desired shelf life of the bag 10 and its contents, the considered gas(es), and the characteristics of the non-porous portion 24. In one non-limiting example, a low permeability to oxygen is a permeability less than 1,000cm 3< /25µm/24h. The non-porous portion 24 may include one or more layers of polymeric materials, including but not limited to polyethylene, in particular low density polyethylene (LDPE) or linear low density polyethylene (LLDPE), metallocene polyethylene, biaxially-oriented polyethylene terephthalate (BoPET), ethylene-vinyl acetate/polyethylene (EVA/PE), and/or mixtures thereof.

The porous portion 26 may be adapted or configured to permit gas sterilization therethrough during a particular sterilization process, while remaining impermeable to contaminants including microorganisms, bacteria, etc. Such gas sterilization may include, for example, transmission or exposure to ethylene oxide (EtO) and/or steam through the porous portion 26 under desired or selected pressure cycles, vacuum strength, and/or time duration. By porous, it is meant that the material, thickness, surface area, and permeability to gas of the porous portion 26 are configured to allow a sufficient flow of sterilizing gas to pass through the porous portion 26. In one example, the porous portion 26 may be constructed from a nonwoven material made of high-density polyethylene (HDPE) fibers, such as Tyvek. In other examples, the nonwoven material may at least partially include coated cellulose fibers and/or uncoated cellulose fibers.

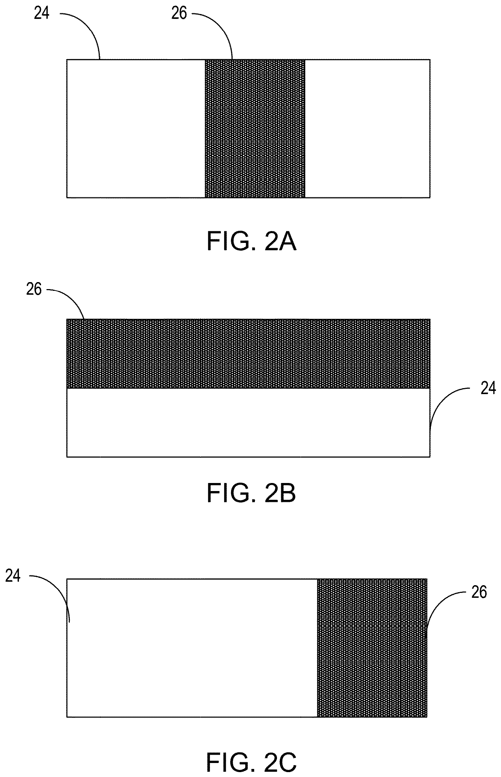

The porous portion 26 may comprise, constitute, or otherwise include a shaped segment that is inserted into, welded, or otherwise coupled to the non-porous portion 24. For example, in the illustrated example shown in FIG. 1, the porous portion 26 may include a round segment centered in or otherwise surrounded by the non-porous portion 24. In another non-limiting example as shown in FIG. 2A, the porous portion 26 may be attached, welded, or otherwise disposed between two separate portions of to a first edge of the non-porous portion 24.

In other non-limiting examples, the porous portion 26 may comprise, constitute, or otherwise include a segment attached to an outer edge of the non-porous portion 24 and having the same length and/or width as the non-porous portion 24, as shown in FIGS. 2B-2C. The bag 10 may include a second sheet 28 of flexible material at least partially attached to the first sheet 12. The second sheet 28 may include or define a first surface 30, a second surface 32 opposite the first surface 30, a first end 34, and a second end 36 opposite the first end 34 in a direction along the longitudinal axis 22. The second sheet 28 may define a length L2 as measured between the first and second ends 34, 36 along the longitudinal axis 22. The length L2 may be less than the length L1 of the first sheet 12.

The second sheet 28 may be substantially non-porous and gas-impervious. The second sheet 28 may be constructed from or otherwise include one or more layers of polymeric materials, including but not limited to polyethylene, such as low density polyethylene (LDPE) or linear low density polyethylene (LLDPE), metallocene polyethylene, biaxially-oriented polyethylene terephthalate (BoPET), ethylene-vinyl acetate/polyethylene (EVA/PE), and/or mixtures thereof.

The second sheet 28 may be attached to the first surface 14 of the first sheet 12 such that a first compartment 38 is formed or defined between the first surface 14 of the first sheet 12 and the first surface 30 of the second sheet 28. The second sheet 28 may be sealed, adhered, fused, or otherwise connected to the first sheet 12 to provide the first compartment 38. For example, the second end 36 of the second sheet 28 may form a first seal 40 with the first sheet 12 adjacent to or in proximity to the second end 20 of the first sheet 12. The first seal 40 may extend along a width of the first and/or second sheets 12, 28, and/or may extend in a direction substantially transverse to the axis 22. The sides of the second sheet 28 may form one or more seals 42 with the respective sides of the first sheet 12 and extend along at least a portion of the lengths of the first and/or second sheets 12, 28. The one or more seals 42 may extend in a direction substantially parallel to the axis 22. The sealing or attachment between the first sheet 12 and the second sheet 28 may constitute or include a single, contiguous leak-proof seal around the identified portions of the first and second sheets 12, 28 that substantially reduce or prevent fluid flow therethrough.

The first end 34 of the second sheet 28 may be unattached or not sealed to the first sheet 12 to define a first opening 44 between the first and second sheets 12, 28 adjacent to or in proximity to the first end 18 of the first sheet 12, with the first opening 44 providing access to the first compartment 38.

The bag 10 may include a third sheet 46 of flexible material at least partially attached to the first sheet 12. The third sheet 46 may include or define a first surface 48, a second surface 50 opposite the first surface 48, a first end 52, and a second end 54 opposite the first end 52 in a direction along the longitudinal axis 22. The third sheet 46 may define a length L3 as measured between the first and second ends 52, 54 along the longitudinal axis 22. The length L3 may be less than the length L1 of the first sheet 12. The length L3 may be the same as or substantially similar to the length L2 of the second sheet 28.

The third sheet 46 may be substantially non-porous and gas-impervious. The third sheet 46 may be constructed from or otherwise include one or more layers of polymeric materials, including but not limited to polyethylene, such as low density polyethylene (LDPE) or linear low density polyethylene (LLDPE), metallocene polyethylene, biaxially-oriented polyethylene terephthalate (BoPET), ethylene-vinyl acetate/polyethylene (EVA/PE), and/or mixtures thereof.

The third sheet 46 may be attached to the second surface 16 of the first sheet 12 such that a second compartment 56 is formed or defined between the second surface 16 of the first sheet 12 and the first surface 48 of the third sheet 46. The third sheet 46 may be sealed, adhered, fused, or otherwise connected to the first sheet 12 to provide the second compartment 56. For example, the first end 52 of the third sheet 46 may form a seal 58 with the first sheet 12 adjacent to or in proximity to the first end 18 of the first sheet 12. The seal 58 may extend along a width of the first and/or third sheets 12, 46, and/or may extend in a direction substantially transverse to the axis 22. The sides of the third sheet 46 may form one or more seals 60 with the respective sides of the first sheet 12 and extend along at least a portion of the lengths of the first and/or third sheets 12, 46. The one or more seals 60 may extend in a direction substantially parallel to the axis 22. The sealing or attachment between the first sheet 12 and the third sheet 46 may constitute or include a single, contiguous leak-proof seal around the identified portions of the first and third sheets 12, 46 that substantially reduce or prevent fluid flow therethrough.

The second end 54 of the third sheet 46 may be unattached or not sealed to the first sheet 12 to define a second opening 62 between the first and third sheets 12, 46 adjacent to or in proximity to the second end 20 of the first sheet 12, with the second opening 62 providing access to the second compartment 56.

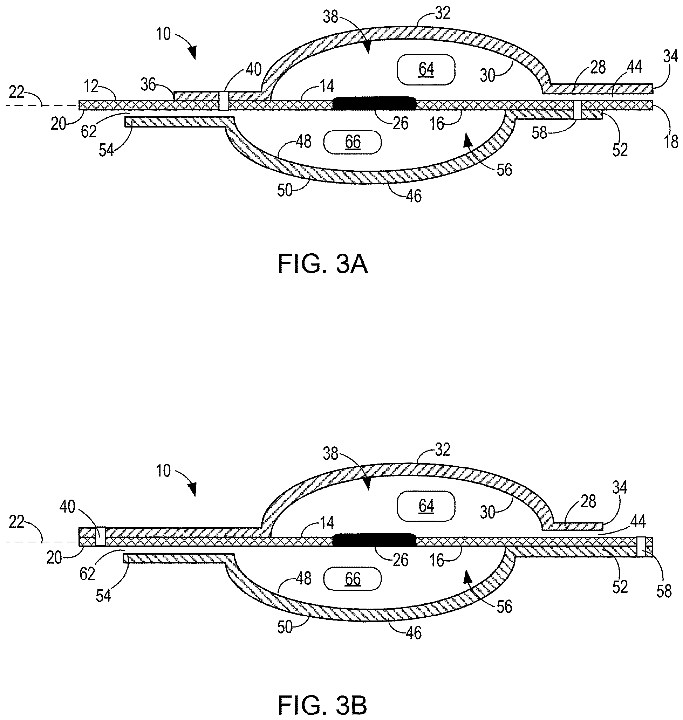

The second sheet 28 and the third sheet 46 may be attached to the first sheet 12 such that the first compartment 38 is longitudinally offset from the second compartment 56. For example, as shown in FIG. 3A, the first opening 44 of the first compartment 38 between the first and second sheets 12, 28 may be closer to the first end 18 of the first sheet 12 than the seal 58 between the first and third sheets 12, 46 forming a boundary or perimeter of the second compartment 56. Additionally and/or alternatively, the second opening 62 of the second compartment 56 between the first and third sheets 12, 46 may be closer to the second end 20 of the first sheet 12 than the seal 40 between the first and second sheets 12, 28 forming a boundary or perimeter of the first compartment 38. In an alternative example as shown in FIG. 3B, the first seal 40 of the first compartment 38 may be laterally positioned beyond the second end 54 of the third sheet 46, and the first seal 58 of the second compartment 56 may be laterally positioned beyond the first end 34 of the second sheet 28 to form the offset compartments.

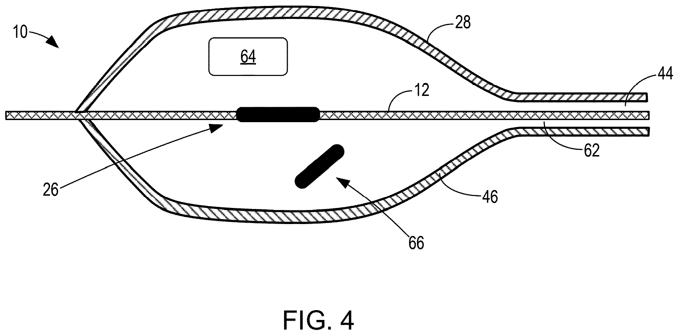

In one non-limiting example, the first opening 44 of the first compartment 38 and the second opening 62 of the second compartment 56 may both be oriented or positioned adjacent to or in proximity of the same end of the first sheet 12. For example, as shown in FIG. 4, the first opening 44 of the first compartment 38 and the second opening 62 of the second compartment 56 may both be oriented or positioned adjacent to or in proximity of the second end 20 of the first sheet 12. The first opening 44 and the second opening 62 may be longitudinally offset from each other or may alternatively be substantially longitudinally aligned. The seal 40 and the seal 58 may both be oriented or positioned adjacent to or in proximity of the same end of the first sheet 12, opposite the end of the first sheet 12 where the first and second openings 44, 62 are positioned. The seal 40 and the seal 58 may be longitudinally offset from each other or may alternatively be substantially longitudinally aligned.

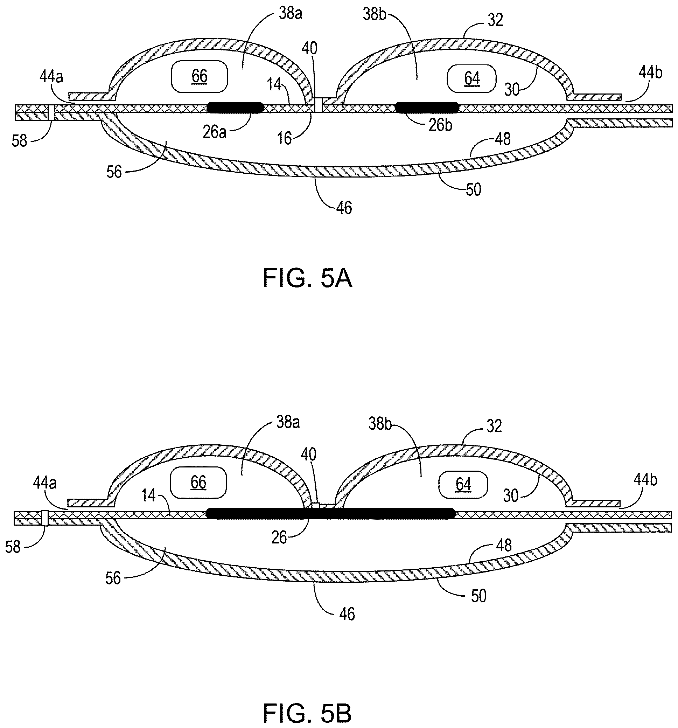

In one non-limiting example, the second sheet 28 of the bag 10 may be sealed, coupled, or otherwise attached to the first sheet 12 to create or define multiple compartments between the first surface 14 of the first sheet 12 and the first surface 30 of the second sheet 28. For example, as shown in FIG. 5A, the second sheet 28 may be attached or coupled to the first sheet 12 to define a first subcompartment 38a and a second subcompartment 38b with respective openings 44a, 44b. The bag 10 may include a first porous portion 26a between the first subcompartment 38a and the second compartment 56, and may include a second porous portion 26b between the second subcompartment 38b and the second compartment 56. In an alternative configuration, the bag 10 may include a single porous portion 26a that extends between both the first subcompartment 38a and the second subcompartment 38b to provide porosity with the second compartment 56, as shown in FIG. 5B. One or more devices to be sterilized, desiccants, charcoal elements, or otherwise may be placed in any combination of the three compartments for a particular use.

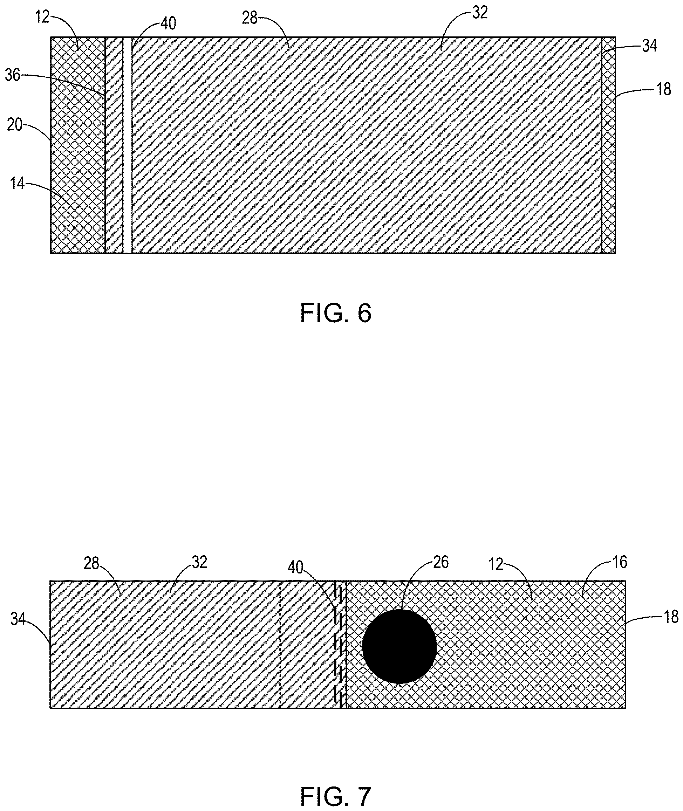

The bag 10 may be assembled and/or manufactured in a variety of different ways to provide the features and benefits disclosed herein. For example, the first, second, and third sheets 12, 28, 46 may initially be separate and unattached from one another. Now referring to FIG. 6, the second sheet 28 may be positioned to overlay or cover at least a portion of the first surface 14 of the first sheet 12. The positioning of the second sheet 28 may be skewed closer to either end of the first sheet 12. For example, the longitudinal distance between the first end 34 of the second sheet 28 and the first end 18 of the first sheet 12 may be less than the longitudinal distance between the second end 36 of the second sheet 28 and the second end 20 of the first sheet 12. The sides of the first sheet 12 and the sides of the second sheet 28 may substantially align. Once the desired positioning between the first and second sheets 12, 28 is achieved, the seal 40 may be formed. The seal 40 may be formed, for example, by thermal fusion or welding or other heating means. Alternatively, one or more adhesives or other sealing mechanisms may be implemented.

Now referring to FIG. 7, after the seal 40 has been formed, the second sheet 28 may be folded about the seal 40 or otherwise repositioned such that first end 34 of the second sheet 28 does not overlap the first sheet 12. The joined first and second sheets 12, 28 may then be turned over such that the second surface 16 of the first sheet 12 is exposed or facing outward.

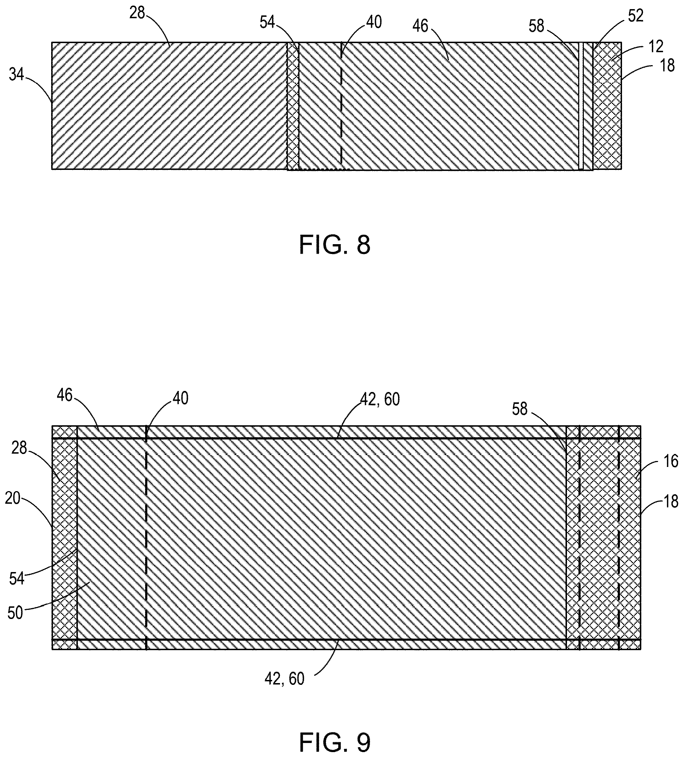

Now referring to FIG. 8, the third sheet 46 may be positioned to overlay or cover at least a portion of the second surface 16 of the first sheet 12. The positioning of the third sheet 46 may be skewed closer to either end of the first sheet 12. For example, the longitudinal distance between the second end 54 of the third sheet 46 and the second end 20 of the first sheet 12 may be less than the longitudinal distance between the first end 52 of the third sheet 46 and the first end 18 of the first sheet 12. The sides of the first sheet 12 and the sides of the third sheet 46 may substantially align. Once the desired positioning between the first and third sheets 12, 46 is achieved, the seal 58 may be formed. The seal 58 may be formed, for example, by thermal fusion or welding or other heating means. Alternatively, one or more adhesives or other sealing mechanisms may be implemented. In the use of a thermal sealing modality, the seal 58 does not penetrate or affect the second sheet 28 due to the prior re-positioning of the second sheet 28.

In this configuration, the seal 40 secures the second end 36 of the second sheet 28 to the first sheet 12, and the seal 58 secures the first end 52 of the third sheet 46 to the first sheet 12. Now referring to FIG. 9, after the seal 58 has been formed, the second sheet 28 may be repositioned such that the first, second, and third sheets 12, 28, 46 all overlap each other. Once the desired positioning between the first, second, and third sheets 12, 28, 46 is achieved, the seals 42, 60 may be formed. The seals 42, 60 may be formed, for example, by thermal fusion, welding, or other heating means. Alternatively, one or more adhesives or other sealing mechanisms may be implemented. In the use of a thermal sealing modality, the seals 42, 60 may be formed through all three sheets 12, 28, 46 in a single pass or procedure along the lengths of the sheets 12, 28, 46 and thus thermally penetrate and effectively seal the overlaid portions or sides of the first, second, and third sheets 12, 28, 46. Once the seals 42, 60 are formed, the first compartment 38 and second compartment 56 are formed and are accessible through the first and second openings 44, 62, as described herein.

In one example of assembly of the bag 10 as shown in FIG. 3B, the third sheet 46 may be aligned underneath the first sheet 12 and the second sheet 28 may be positioned on top of the second sheet 28. The first end 52 of the third sheet 46 may be positioned closer to the first end 18 of the first sheet 12 than the first end 34 of the second sheet 28. The second end 36 of the second sheet 28 may be positioned closer to the second end 20 of the first sheet 12 than the second end 54 of the third sheet 46. The seals 40, 58 may then be formed by a single thermal application directly through all three sheets without needing to fold or re-position any of the sheets, since the second sheet 28 does not overlap with the seal 58 and the third sheet 46 does not overlap with the seal 40.

In an example of use of the bag 10, one or more items 64 to be sterilized may be inserted through the first opening 44 of the first compartment 38. The one or more items 64 may include, for example, one or more medical devices, instruments, and/or components thereof. Once the desired items 64 are within the first compartment 38, the first opening 44 may be sealed (e.g., by sealing the respective portion of the second sheet 28 to the first sheet 12) by thermal, adhesive, or other means. One or more sterilizing agents may then be introduced through the second opening 62 of the second compartment 56, such that the one or more sterilizing agents permeates or otherwise passes into the first compartment 38 through the porous portion 26 of the first sheet 12 to sterilize the items 64 in the first compartment 38. The sterilizing agent may include, for example, ethylene oxide or other effective compounds.

Once the sterilization process is complete, one or more charcoal elements 66, which may include activated charcoal and/or other additives, may be placed into the second compartment 56. In addition and/or alternatively to the charcoal elements 66, one or more desiccant elements (not shown) may be positioned in the second compartment 56. The one or more charcoal elements 66 may aid in absorbing excess sterilizing agent, residual moisture, or the like remaining in the bag 10 after the sterilization process. A vacuum may be created or established within the first and second compartments 38, 56, and the second opening 62 of the second compartment 56 may be sealed (e.g., by sealing the respective portion of the third sheet 46 to the first sheet 12) by thermal, adhesive, or other means creating a visual sterility maintenance indicator. The sterilized items 64 in the bag 10 may then be stored, transported, etc., until ready for use.

When ready for use, the first compartment 38 may be opened individually and independently of the second compartment 56 to access and use the items 64, while the charcoal element 66 remains secured in the second compartment 56. The bag 10 may then be disposed of with the charcoal element 66 secured therein and/or otherwise processed for re-use or other functionality.

It will be appreciated by persons skilled in the art that the present disclosure is not limited to what has been particularly shown and described herein above. In addition, unless mention was made above to the contrary, it should be noted that all of the accompanying drawings are not to scale. Of note, the system components have been represented where appropriate by conventional symbols in the drawings, showing only those specific details that are pertinent to understanding the embodiments of the present disclosure so as not to obscure the disclosure with details that will be readily apparent to those of ordinary skill in the art having the benefit of the description herein. Moreover, while certain embodiments or figures described herein may illustrate features not expressly indicated on other figures or embodiments, it is understood that the features and components of the examples disclosed herein are not necessarily exclusive of each other and may be included in a variety of different combinations or configurations without departing from the scope and spirit of the disclosure. A variety of modifications and variations are possible in light of the above teachings without departing from the scope and spirit of the disclosure, which is limited only by the following claims.

## Claims

1. A bag, comprising:
a first sheet including a porous portion and a gas-impervious portion, the first sheet having:
a first surface,
a second surface opposite the first surface,
a first end,
a second end opposite the first end, and
a longitudinal axis extending between the first and second ends;
a second sheet attached to the first surface of the first sheet to define a first compartment between the first and second sheets; and
a third sheet attached to the second surface of the first sheet to define a second compartment between the first and third sheets, wherein the second compartment is longitudinally offset from the first compartment.

2. The bag of claim 1, further comprising a first opening between the first and second sheets, wherein the first opening is located adjacent the first end of the first sheet and in fluid communication with the first compartment.

3. The bag of claim 2, further comprising a second opening between the first and third sheets, wherein the second opening is located adjacent the second end of the first sheet and in fluid communication with the second compartment.

4. The bag of claim 3, further comprising a first seal joining the first sheet and the second sheet, wherein the first seal extends substantially transverse to the longitudinal axis and is longitudinally positioned between the first opening and the second end of the first sheet.

5. The bag of claim 4, wherein the second opening is longitudinally positioned between the first seal and the second end of the first sheet.

6. The bag of claim 4, further comprising a second seal joining the first sheet and the third sheet, wherein the second seal extends substantially transverse to the longitudinal axis and is longitudinally positioned between the second opening and the first end of the first sheet.

7. The bag of claim 6, wherein the first opening is longitudinally positioned between the second seal and the first end of the first sheet.

8. The bag of claim 1, wherein the first sheet defines a length between the first end and second end, and wherein the length is greater than a length of the second sheet, and optionally the length of the first sheet is greater than a length of the third sheet.

9. A method of sterilizing an article, comprising:
providing the bag of any of claims 1-8;
placing an item in the first compartment of the bag;
sealing the second sheet to the first sheet;
introducing a sterilizing agent into the second compartment such that the sterilizing agent passes through the porous portion of the first sheet and into the first compartment;
positioning a charcoal element into the second compartment; and
sealing the third sheet to the first sheet.

10. The method of claim 9, further comprising creating a vacuum in the second compartment prior to sealing the third sheet to the first sheet.

11. The method of claim 9, wherein the sterilizing agent is at least one of ethylene oxide or steam.

12. A method of assembling a bag, comprising:
positioning a first sheet in contact with least a portion of a second sheet, wherein the first sheet includes a porous portion and a gas-impervious portion, the first sheet further including:
a first surface,
a second surface opposite the first surface,
a first end,
a second end opposite the first end, and
a longitudinal axis extending between the first and second ends;
forming a first seal between the first surface of the first sheet and the second sheet, wherein the first seal extends substantially transverse to the longitudinal axis;
moving the second sheet such that at least a portion of the second sheet does not overlap the first sheet;
positioning a third sheet in contact with at least a portion of the second surface of the first sheet second sheet;
forming a second seal between the first surface of the first sheet and the second sheet, wherein the first seal extends substantially transverse to the longitudinal axis;
positioning the first sheet, second sheet, and third sheet to substantially overlay each other;
forming a third seal between the first, second, and third sheets, wherein the third seal extends substantially parallel to the longitudinal axis; and
forming a fourth seal between the first, second, and third sheets, wherein the fourth seal extends substantially parallel to the longitudinal axis, wherein the first, third, and fourth seals define a first compartment between the first and second sheets, and wherein the second, third, and fourth seals define a second compartment between the first and third sheets.

13. The method of claim 12, wherein the first compartment is longitudinally offset from the second compartment, and optionally, the first compartment is accessible through a first opening between the first and second sheets adjacent to the first end of the first sheet, and optionally the second compartment is accessible through a second opening between the first and third sheets adjacent to the second end of the first sheet.

14. The method of claim 12, wherein the first seal is longitudinally positioned between the second opening and the second seal, and optionally the second seal is longitudinally positioned between the first opening and the first seal.

15. The method of claim 12, wherein the first sheet defines a length between the first end and second end, and wherein the length is greater than a length of the second sheet, and optionally the length of the first sheet is greater than a length of the third sheet.
